# EUROPÄISCHE PATENTSCHRIFT

(11) **EP 1 056 448 B1**
(45) Veröffentlichungstag und Bekanntmachung des Hinweises auf die Patenterteilung: **14.01.2004**
(21) Anmeldenummer: 99916744.8
(22) Anmeldetag: 17.02.1999
(51) Int. Cl.: A61K 31/135, A61K 31/165, A61K 31/415, A61P 25/06

(54) **PHARMAZEUTISCHE KOMBINATIONEN MIT TRAMADOL**
PHARMACEUTICAL COMBINATIONS CONTAINING TRAMADOL
COMBINAISONS PHARMACEUTIQUES CONTENANT DU TRAMADOL

(30) Priorität: 21.02.1998 DE 19807535
(43) Veröffentlichungstag der Anmeldung: 06.12.2000
(73) Patentinhaber: VIATRIS GmbH & Co. KG, 60314 Frankfurt am Main (DE)
(72) Erfinder: RABER, Marc, D-35390 Giessen (DE); MOMBERGER, Helmut, D-35037 Marburg (DE)
(86) Internationale Anmeldenummer: PCT/DE1999/000428
(87) Internationale Veröffentlichungsnummer: WO 1999/042095

(56) Entgegenhaltungen:
- EP-A- 0 011 490
- EP-A- 0 774 253
- WO-A-97/18801
- GB-A- 2 313 309
- LEHMANN K A: "TRAMADOL FOR THE MANAGEMENT OF ACUTE PAIN" DRUGS, Bd. 47, Nr. SUPPL. 01, 1. Januar 1994 (1994-01-01), Seiten 19-32, XP002061725 ISSN: 0012-6667
- DATABASE MEDLINE [Online] US NATIONAL LIBRARY OF MEDICINE (NLM), BETHESDA, MD, US LEHMANN K A: "[ Tramadol in acute pain]. Le tramadol dans les douleurs aigues." retrieved from STN Database accession no. 97274803 XP002110372 & DRUGS, (1997) 53 SUPPL 2 25-33. REF: 32 ,
- DATABASE BIOSIS [Online] BIOSCIENCES INFORMATION SERVICE, PHILADELPHIA, PA, US KAINZ C ET AL: "EFFICACY AND TOLERANCE OF TRAMADOL WITH OR WITHOUT ANTIEMETIC COMPARED TO PETHIDINE IN OBSTETRIC ANALGESIA." retrieved from STN XP002110374 & Z GEBURTSHILFE PERINATOL, (1992) 196 (2), 78-82. ,
- DATABASE EMBASE [Online] ELSEVIER SCIENCE PUBLISHERS, AMSTERDAM, NL Database accession no. 97345409, NG K. F. J. ET AL: "Comparison of tramadol and tramadol/droperidol mixture for patient-controlled analgesia" XP002110373

## Beschreibung

Die vorliegende Erfindung betrifft die Verwendung pharmazeutischer Kombinationen, die als Wirkstoffe Tramadol oder eines seiner Enantiomere oder eines seiner pharmazeutisch akzeptablen Salze und Metoclopramid, Domperidon oder andere prokinetisch und antiemetisch wirksame Substanzen oder eines ihrer pharmazeutisch akzeptablen Salze enthalten, zur Behandlung der Migräne und migräniformer Kopfschmerzen sowie zur Behandlung von Schmerzzuständen, die mit Übelkeit und/oder Erbrechen (beispielsweise unter Chemotherapie) und/oder einer verzögerten Magenentleerung einhergehen (beispielsweise postoperativ, bei diabetischer Gastroparese).

Die Migräne ist eine Erkrankung mit wiederkehrenden Kopfschmerzattacken, die zwischen 4-72 Stunden anhalten. Migräneattacken sind überwiegend einseitige, dumpf beginnende, dann pulsierende Kopfschmerzen von mittlerer bis starker Intensität. Typische Begleitsymptome der Migräne sind Licht- und Geräuschüberempfindlichkeit, Gesichtsblässe, Übelkeit und Erbrechen mit und ohne neurologische Herdausfälle als Prodromalstadium.

Die (gewöhnliche) Migräne ohne Aura wird von der (klassischen) Migräne mit Aura unterschieden, die jeweils mit einem charakteristischen Flimmerskotom beginnt. Eine komplizierte Migräne liegt vor, wenn die Sehstörungen über Tage anhalten oder andere neurologische Herdsymptome auftreten mit den bekannten Sonderformen der retinalen, basilären, ophthalmoplegischen, aphasischen oder hemiplegischen Migräne.

Es existieren verschiedene Vorstellungen über den Pathomechanismus der Migräne. Die frühere hämodynamische Vorstellung, nach der die initialen neurologischen Ausfälle durch regionale intrakranielle Vasokonstriktion und der nachfolgende pulsierende Kopfschmerz durch extrakranielle Vasodilatation mit Schmerzleitung über den Nervus ophthalmicus und Nervus trigeminus ausgelöst werden, erklärt die Vorgänge bei der Migräne nur unzureichend.

Die regionale Hirndurchblutung ist bei einer Migräneattacke mit Aura okzipital vermindert, wobei die langsame Wanderung der kortikalen Oligamie mit Überschreitung der Versorgungsbereiche einzelner Arterien dafür spricht, daß nicht nur vasomotorische, sondern auch elektrophysiologische Phänomene entsprechend der sogenannten "spreading depression" beteiligt sind (Spierings ELH (1988); Recent advances in the understanding of migraine. Headache 28: 655-658).

Andere Befunde sprechen dafür, daß der begleitende Kopfschmerz nicht nur durch eine extrakranielle Vasodilatation, sondern auch durch eine zentrale Erniedrigung der Schmerzschwelle ausgelöst wird, wobei in den Zellen des Rückenmarks und des Hirnstammes nach noxischer Reizung sog. schnellinduzierbare Gene (IEG's immediate early genes) aktiviert werden (Zimmermann, M. (1995); Neurobiologie des Schmerzsystems. Neuroforum 1/95: 34-45).

Eine andere Theorie - das Modell der neurogenen Entzündung - bietet die Möglichkeit sowohl die Blutflußveränderung als auch die erhöhte Schmerzempfindlichkeit der Gefäße während der Migräneattacke zu erklären. Demnach wird die erhöhte Schmerzempfindlichkeit durch eine verstärkte Sensibilisierung der sensorischen perivaskulären Fasern des trigeminovaskulären Systems hervorgerufen. Durch diese erhöhte Sensibilisierung sind Gefäßpulsationen, die normalerweise nicht in der Lage sind, schmerzhafte Empfindungen auszulösen, potente Schmerzreize und bedingen den pulsierenden pochenden Migräneschmerz. Die neurogene Entzündung wird ausgelöst durch noxische Stimulation der perivaskulären Nervenfasern der meningealen Blutgefäße. Aus den Nervenenden, die wahrscheinlich gleichzeitig Nozizeptoren sind, werden Neuropeptide wie Substanz P, Neurokinin A und CGRP (calcitonin-gene related peptide) ausgeschüttet, die in der Lage sind, die neurogene Entzündung auszulösen. CGRP läßt sich während eines Migräneanfalles auch vermehrt im venösen Blut des Kopfes nachweisen.

Durch Ausschüttung der Neuropeptide wird ein circulus vitiosus in Gang gesetzt: Peptidfreisetzung - Vasodilation und kapilläre Permeabilitätssteigerung - vermehrte Erregung von Nozizeptoren - vermehrte Peptidfreisetzung. Die Wirkstoffe Sumatriptan und Ergotalkaloide inhibieren die Freisetzung der Neuropeptide und unterbrechen so den schmerzauslösenden Kreislauf (Zimmermann, M.: Chronische Schmerzen und ihre Ursachen, Deutsches Ärzteblatt 93, Heft 43, 1996: A-2749-2752).
Andere Befunde sprechen für eine primär neurogene hypothalmische Dysfunktion, wobei dem vasoaktiven Serotonin, das während des Migräneanfalles aus den Raphekernen des Hirnstammes vermindert ausgeschüttet wird, eine Schlüsselrolle zukommt (Ferrari MD et al. (1989): Serotonin metabolism in migraine. Neurology 39: 1239-1242; Pramod R. et al. (1989): 5-HT₁-like receptor agonists and the pathophysiology of migraine, Trends in Pharmacological Sciences 10, 200-204).

Ungeachtet vieler Hypothesen und komplizierter Modellvorstellungen ist der Pathomechanismus der Migräne bislang nicht verstanden. Die Migräne hat eine multifaktorielle Genese mit genetischer Disposition, externen (wie Alkohol) und internen (wie Hormone) Triggermechanismen. Es handelt sich nicht um eine psychosomatische Erkrankung, obwohl psychische Faktoren eine Attacke auslösen können.

Zur Behandlung der Migräne sind eine Vielzahl von Präparaten, die einzelne oder mehrere Wirkstoffe in fixer Kombination enthalten, bekannt.
Im allgemeinen werden zur Behandlung leichter Migräneattacken Antiemetika wie Metoclopramid oder Domperidon allein oder in Kombination mit Nicht-Opioid-Analgetika wie Acetylsalicylsäure, Paracetamol, Ibuprofen oder Naproxen empfohlen. Bei mittelschweren bis schweren Migräneattacken sind Antiemetika in Kombination mit Mutterkornalkaloiden wie Ergotamin oder Dihydroergotamin oder auch Sumatriptan Mittel der Wahl.
Häufige Nebenwirkungen von Ergotamin und Dihydroergotamin sind Übelkeit, Brechreiz, Erbrechen, Kopfschmerzen. Muskelschmerzen und ein allgemeines Kältegefühl, Symptome also, die unter der falschen Annahme einer fortgesetzten Migräneattacke zur wiederholten Einnahme solcher Präparate und damit zur Überdosierung führen können. Durch häufige Anwendung können Dauerkopfschmerzen entstehen,die einem Ergotaminabusus Vorschub leisten. An schwerwiegenden Nebenwirkungen können Durchblutungsstörungen, koronare Herzkrankheit (KHK), arterielle Verschlußkrankheit (AVK), Hypertonie und pektanginöse Beschwerden auftreten. In der Schwangerschaft und Stillzeit sowie bei Kindern unter 12 Jahren dürfen Ergotalkaloide nicht angewendet werden.

Sumatriptan und seine Derivate (Almotriptan, Eletriptan, Naratriptan, Rizatriptan, Zolmitriptan) sind sehr wirksame Migränemittel, die bei oraler Anwendung den Einzelsubstanzen Ergotamin, Acetylsalicylsäure oder Metoclopramid überlegen sind. Sumatriptan ist bei Kindern, in der Schwangerschaft und Stillzeit, bei Patienten über 65 Jahre und bei Patienten mit koronarer Herzkrankheit kontraindiziert. An unerwünschten Wirkungen können auftreten Druck- und Wärmegefühl, allgemeines Schwächegefühl, Engegefühl in der Brust. Hypertonie, koronare Herzkrankheit (KHK), Myokardinfarkt, Angina pectoris.

Da bei der Migräne die Magenentleerung gehemmt oder erheblich verlangsamt ist und der Magen als Resorptionsort von Pharmaka wegen der relativ kleinen Resorptionsfläche und der im Vergleich zum Dünndarm wesentlich geringeren Vaskularisation von untergeordneter Bedeutung ist, kommt es bei einer Migräneattacke nur zu einer unzureichenden oder zeitlich stark verzögerten Resorption des Analgetikums. Für die Schnelligkeit des Wirkungseintritts des Schmerzmittels spielt daher die Entleerungszeit des Magens eine große Rolle.

Durch Prokinetika wie Metoclopramid und Domperidon kann die Magenentleerung und damit die Resorption des Schmerzmittels beschleunigt werden.

Metoclopramid und Domperidon haben ihre Berechtigung in der Migränetherapie aber nicht nur wegen ihrer prokinetischen Wirkungen, sondern auch wegen ihrer Wirkung gegen Symptome wie Übelkeit und Erbrechen, die häufig als Begleitsymptome bei der Migräne vorkommen.

Die medikamentöse Therapie der Migräne ist symptomatischer Natur, sie heilt das Leiden nicht. Von Mischpräparaten aus Nichtopioid-Analgetika und Mischpräparaten aus sekalen Alkaloiden wird abgeraten, da sie bei längerem Gebrauch, vor allem bei täglichem Gebrauch, ihrerseits Kopfschmerzen verursachen. Ferner sind Leber- und Nierenschäden, wie die sogenannte Analgetika-Nephropathie, mögliche Folgen der langfristigen Einnahme von Analgetikakombinationen.
Opioid-Analgetika sind im allgemeinen wegen ihres Mißbrauchs- und Abhängigkeitspotentials zur Therapie der Migräne nicht geeignet.

Aus der Patentliteratur sind zur Behandlung von Migräne insbesondere folgende Kombinationen bekannt:
- Paracetamol und Metoclopramid (EP 011 489, EP 011 490, US 5 437 874, EP 695 546, EP 774 253)
- Acetylsalicylsäure oder das L-Lysinacetylsalicylat davon und Metoclopramid (EP 606 031)
- Analgetikum (wie Acetylsalicylsäure), Antiemetikum (wie Metoclopramid) und ein Antacidum (CA 20 20 018)

Die oben in der Patentliteratur aufgeführten Analgetikakombinationen eignen sich im allgemeinen zur Behandlung leichter Migräneattacken. Zur Behandlung der mittelschweren bis schweren Migräne sind sie nicht geeignet. Bei der schweren Migräne sind im allgemeinen Ergotalkaloide in Kombination mit einem Antiemetikum oder Sumatriptan indiziert.

Nachteilig ist, daß unter der Therapie mit Ergotalkaloiden oder Sumatriptan eine Vielzahl an teilweise schweren kardiovaskulären Nebenwirkungen wie Angina pectoris, koronare Herzkrankheit (KHK),Hypertonie, und Myokardinfarkt auftreten können.

Es besteht daher nach wie vor ein großer Bedarf nach einem zuverlässigen, bei mäßigstarken bis starken Migräneattacken gut wirkenden Schmerzmittel mit wenig Nebenwirkungen.

Aufgabe der vorliegenden Erfindung ist deshalb insbesondere die Bereitstellung eines verbesserten Therapeutikums zur Behandlung der mittelschweren bis schweren Migräneattacke.

Die Erfindung betrifft somit den in den Patentansprüchen beanspruchten Gegenstand.

Tramadol (1RS;2RS)-2[(Dimethyl-amino)methyl]-1-(3-methoxyphenyl)-cyclohexanol ist ein Analgetikum, das bei mäßig starken bis starken Schmerzen wirksam ist.
Tramadol gehört zur Gruppe der schwach wirksamen Opioide. Tramadol zeichnet sich im Vergleich zu anderen Opioiden durch eine geringere Toleranzentwicklung hinsichtlich der analgetischen Wirkung, durch das weitgehende Fehlen opiattypischer Nebenwirkungen, als auch durch ein sehr geringes Abhängigkeitspotential aus.
Die analgetische Wirkung von Tramadol schließt sowohl opioide als auch nicht-opioide Komponenten ein, letztere über die Freisetzung von Serotonin (5-HT) und Hemmung der Wiederaufnahme von Serotonin und Noradrenalin im zentralen Nervensystem (ZNS).
Einen wesentlichen Beitrag zur analgetischen Wirkung von Tramadol leisten die nicht-opioiden Wirkkomponenten.

Die Noradrenalin-Wiederaufnahme wird überwiegend durch das (-)-Enantiomer gehemmt und die Serotonin-Freisetzung sowie die Hemmung der Wiederaufnahme von Serotonin aus dem synaptischen Spalt wird maßgeblich durch das (+)-Enantiomer hervorgerufen. Beide Enantiomere tragen zur analgetischen Wirkung am Menschen bei.

Unter der Behandlung mit Tramadol können an unerwünschten Wirkungen gelegentlich Übelkeit, Erbrechen, Schwitzen, Mundtrockenheit, Schwindel und Benommenheit auftreten. Selten werden gastrointestinale Beschwerden oder verschiedenartige psychische Nebenwirkungen beobachtet.

Prokinetisch wirksame Verbindungen, deren Hauptvertreter Metoclopramid und Domperidon sind, erhöhen den Tonus des unteren Ösophagussphincters und beschleunigen die Magenentleerung und Dünndarmpassage.
Gleichzeitig werden diese Wirkstoffe als Antiemetika, das heißt zur Unterdrückung von Übelkeit, Brechreiz und Erbrechen eingesetzt.
Metoclopramid und Domperidon sind indiziert bei der Gastroparese, die postoperativ und bei einigen Grunderkrankungen auftreten kann (z.B. Diabetes mellitus u.a.). Sie werden auch bei der funktionellen Dyspepsie (Reizmagen) gegeben, als deren Ursache Störungen der gastrointestinalen Motilität vermutet werden.
Ferner sind sie indiziert bei Migräne und anderen Schmerzerkrankungen, die mit Störungen der Magenentleerung einhergehen.

Überraschenderweise wurde festgestellt, daß mit der Verabreichung von Tramadol in Kombination mit prokinetisch wirksamen Antiemetika ein verbessertes Mittel zur Behandlung insbesondere von mittelschweren bis schweren Migräneattacken zur Verfügung steht.

Opioide sind zur Behandlung der Migräne im allgemeinen nicht geeignet, da sie mit einer Vielzahl von Nebenwirkungen belastet sind und zu psychischer und physischer Abhängigkeit führen können.

Darüber hinaus wirken Opioide häufig obstipierend, wodurch die der Migräne zugrundeliegende Magenatonie verstärkt und damit die Resorption und der Wirkungseintritt des Schmerzmittels verzögert wird.

Eine Ausnahme unter den Opioid-Analgetika bildet Tramadol, das nicht diese schwerwiegenden Nebenwirkungen zeigt, die typischerweise bei der Anwendung von Opioiden gesehen werden.

Überraschend wurde auch festgestellt, daß durch die erfindungsgemäße Kombination aus Tramadol und insbesondere Metoclopramid die Migräneattacke wirksam kupiert und das Auftreten der Symptome Übelkeit und Erbrechen verhindert werden kann.

Das war umso überraschender, als Tramadol selbst Übelkeit und Erbrechen auslösen bzw. diese Begleitsymptome der Migräne verstärken kann.
Deshalb wurde Tramadol als Monopräparat zur Migränebehandlung in der Vergangenheit als ungeeignet angesehen.

Durch die Kombination mit Metoclopramid ist es möglich, das bei mäßig starken und starken Schmerzen gut wirksame Schmerzmittel Tramadol auch zur Migränebehandlung zur Verfügung zu stellen.

Da Metoclopramid bei der Migräne einen eigenen analgetischen Effekt besitzt, wie placebo-kontrollierte Studien belegen, ist zu erwarten, daß bei der gleichzeitigen Gabe von Tramadol und Metoclopramid auch ein synergistischer analgetischer Effekt eintritt.

Darüberhinaus kann durch die fixe Kombination die Verträglichkeit von Tramadol verbessert, die Resorption von Tramadol im Magen-Darm-Trakt beschleunigt und das Auftreten der Begleitsymptome der Migräne Übelkeit und Erbrechen verhindert werden. Auch hinsichtlich ihrer pharmakodynamischen und pharmakokinetischen Eigenschaften sind die Substanzen Tramadol und Metoclopramid bzw. Domperidon als Kombinationspartner in einer fixen pharmazeutischen Zubereitung geeignet. Die analgetische Wirkungsdauer von Tramadol beträgt ca. 4-7 Stunden bei einer terminalen Eliminationshalbwertszeit von ca. 5-6 Stunden. Die prokinetische Wirkungsdauer von Metoclopramid und Domperidon beträgt etwa 1-2 Stunden, die antiemetische etwa 3-5 Stunden. Die Halbwertszeit liegt bei 4-6 Stunden.

Die aufgezeigten Kombinationen sind geeignet zur Behandlung von mäßigen bis starken Migräneschmerzen oder migräneformen Kopfschmerzen.

Außerdem sind diese Kombinationen geeignet zur Behandlung von:
- mäßigen bis starken akuten und chronischen Schmerzen, die mit Übelkeit und/oder Erbrechen einhergehen (z. B. chemotherapie-bedingtes Erbrechen)
- mäßigen bis starken akuten und chronischen Schmerzen bei gleichzeitig vorliegenden Magenentleerungsstörungen (z.B. postoperativ oder bei diabetischer Gastroparese u.a.) und
- bei Übelkeit oder Erbrechen unter Tramadof-Therapie

Anhand von klinischen Versuchen soll die Wirksamkeit der beanspruchten Kombinationen zur Behandlung von Migräne am Beispiel der Kombination Tramadol und Metoclopramid näher erläutert werden.

Es wurden 8 Patienten mit mittelstarken bis starken Migränekopfschmerzen, die sich mit schwach wirksamen Analgetika (z.B. Acetylsalicylsäure, Ibuprofen oder Paracetamol) nicht mehr ausreichend behandeln ließen, in die Anwendungsbeobachtung eingeschlossen.

Die vor Behandlungsbeginn bestehende Stärke der Migränekopfschmerzen und Ausprägung der Begleitsymptome wurde dokumentiert und die Beurteilung der Wirkung im weiteren Behandlungsverlauf durch den Patienten vorgenommen.

Die Schmerzintensität vor und nach Einnahme der Medikamente wurde auf einer 100 mm langen VAS-Skala zum Zeitpunkt 0, 30 min, 1 h, 2 h, 4 h, 6 h und 12 h gemessen. Die Ausprägung der Migränebegleiterscheinungen und deren Veränderung nach Einnahme der Medikamente wurde auf einer mehrstufigen verbalen Rating-Skala dokumentiert. Zu Wirkung und Verträglichkeit konnte der Patient ferner ein Globalurteil abgeben.

Von den 8 behandelten Patienten waren sieben weiblich und einer männlich. Das Alter der Patienten betrug im Mittel 44,7 Jahre (35-63 Jahre). Körpergröße und Körpergewicht lagen im Mittel bei 169,3 cm (159-186 cm) bzw. 70,4 kg (55-103 kg).

Alle Patienten erfüllten die operationalen diagnostischen Kriterien der IHS für die Diagnose Migräne und hatten bereits mindestens 5 Migräneanfälle erlebt, die eine Dauer von 4-72 Stunden aufwiesen. Die Lokalisation der Kopfschmerzen war streng einseitig, die Intensität der Kopfschmerzen wurde durch körperliche Aktivität verstärkt.

Alle Patienten litten während der Kopfschmerzattacken gleichzeitig an Übelkeit und Lärmempfindlichkeit, 28% der Patienten zusätzlich an Erbrechen und 43% an Lichtempfindlichkeit. Die Migräne bestand im Durchschnitt seit 15,3 Jahren (2-38 Jahre); die mittlere Attackenfrequenz lag bei 4,1 Tagen im Monat (2-8 Tage/Monat).

Im Untersuchungszeitraum erlitten die Patienten 11 akute Migräneattacken, die sie mit der freien Kombination Tramadol und Metoclopramid behandelten.

Alle Patienten nahmen die Arzneimittel gleichzeitig in einer oralen Einzeldosis von 100 mg Tramadol (2 Tramadol AWD Kapseln ä 50 mg Tramadol-HCl) und 10 mg Metoclopramid Lösung (30 Tropfen Metoclopramid Temmler 5 mg Lösung) ein.

Vor der dokumentierten Behandlung wurden die Migräneschmerzen von den Patienten als stark bis sehr stark angegeben. Im Mittel betrug der Ausgangsschmerz auf der VAS-Skala 75 ± 7 mm (62-83 mm).

Zwei Patienten, die jeweils zwei Migräneanfälle behandelten, erfuhren unter der Behandlung keine Verbesserung der Schmerzen und wurden als Non-Responder deklariert und aus der Analyse ausgeschlossen.

Übrig blieben 6 Patienten, die 7 Migräneanfälle behandelten. Zur Quantifizierung von Kopfschmerzen wurde in dieser Studie eine 100 mm lange VAS-Skala eingesetzt, welche üblicherweise auch in anderen Schmerzstudien angewendet wird.
Kopfschmerzen wurden dabei grob anhand der folgenden Einteilung bewertet:

| | |
|---|---|
| 0 mm | (keine Kopfschmerzen, **Grad 0),** |
| 1-30 mm | (leichte Kopfschmerzen, **Grad 1),** |
| 31-60 mm | (mittelschwere Kopfschmerzen. **Grad 2),** |
| 61-100 mm | (starke bis sehr starke Kopfschmerzen, **Grad 3).** |

In Migränestudien wird als Erfolgskriterium im allgemeinen meist der Prozentsatz von Patienten angesehen, bei dem es innerhalb eines definierten Zeitraums (meist nach 2 oder 4 Stunden) zu einer Besserung der Kopfschmerzintensität des Grades 3 oder 2 auf 1 oder 0 kommt.

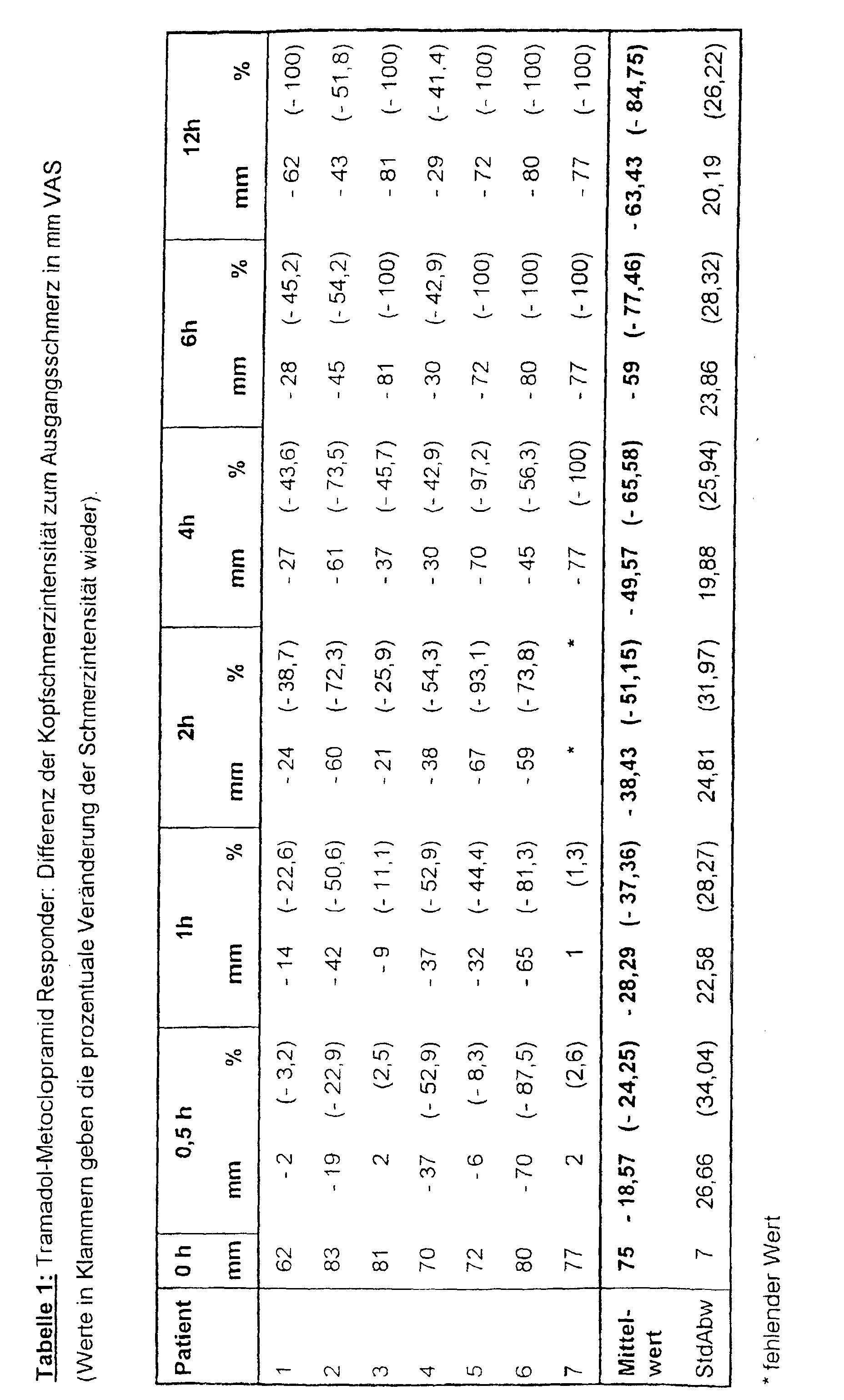

Andererseits haben wir in Anlehnung an oben genannte Konvention der Erfolgs- bzw. Wirksamkeitsbeurteilung von Migränemitteln in Tabelle 2 den Behandlungserfolg zusätzlich als Prozentsatz der Patienten angegeben, der eine Besserung des Kopfschmerzschweregrades von Grad 3 oder 2 auf Grad 1 oder 0 erfuhren (einschließlich Non-Responder).

**Tabelle 2:**

| Besserung der Kopfschmerzintensität von Grad 3 oder 2 auf Grad 1 oder 0 in Abhängigkeit von der Zeit nach oraler Applikation von 100 mg Tramadol Kapseln und 10 mg Metoclopramid-Lösung | | |
|---|---|---|
| **Zeit** | **Anzahl der Patienten** **(n=8)** | **Besserung von Grad 3-2 auf 1-0** **(% Patienten)** |
| 0,5 h | 2 | 25 |
| 1 h | 5 | 62 |
| 2 h | 6 | 75 |
| 4 h | 6 | 75 |
| 6 h | 6 | 75 |
| 12 h | 6 | 75 |

Im Durchschnitt haben sich die Kopfschmerzen nach Einnahme der freien Kombination Tramadol und Metoclopramid in der Gruppe der Responder bereits innerhalb von 30 Minuten um 24,3% und nach 2 Stunden um 51,1 %, bezogen auf den Ausgangsschmerz, verbessert. Nach 4 und 6 Stunden betrug die Reduktion der Schmerzintensität durchschnittlich 65,6% bzw. 77,5% (s. Tabelle 1).

Für die Gesamtgruppe der behandelten Patienten (inkl. der Non-Responder) ergab sich eine Wirksamkeit (Besserung der Kopfschmerzen von stark oder mittelstark auf leicht oder kopfschmerzfrei) von 25% nach 30 Minuten und 75% nach zwei Stunden (s. Tabelle 2).

**Zusammenfassend** läßt sich auch aus den vorliegenden Daten ableiten, daß die Kombination Tramadol und Metoclopramid in der verabreichten Dosierung eine vielversprechende Alternative zu den bisher verfügbaren Migränemitteln, die bei mäßig starken bis starken akuten Migränekopfschmerzen therapeutisch angewendet werden, sein kann.

Diese Kombinationen können in Form von Tabletten, Brausetabletten, Kapseln, Granulaten, Pulvern, Retardtabletten, Retardkapseln (single und multiple unit - Formulierungen), Ampullen zur intravenösen und intramuskulären Injektion und in Form von Infusionslösungen, Suspensionen, Zäpfchen oder in anderen geeigneten Arzneiformen verabreicht werden.

Die Retardarzneiformen können die Wirkstoffe in völlig oder teilweise retardierter Form mit oder ohne Initialdosis-Anteil enthalten.

Die Wirkstoffe können gemeinsam oder in teilweise oder vollständig voneinander getrennten Formulierungen vorliegen, so daß auch eine getrennte oder auch zeitlich abgestufte Verabreichung möglich ist.

Falls solche vollständig getrennten Formulierungen vorliegen, sind diese aufeinander abgestimmt und enthalten die jeweiligen Wirkstoffe in der Dosierungseinheit in denselben Mengen und entsprechenden Gewichtsverhältnissen, in denen sie in der kombinierten Mischung vorliegen können.
In diesen pharmazeutischen Zusammensetzungen können die Wirkstoffe auch in Form ihrer pharmazeutisch verwendbaren Salze vorliegen.

Bevorzugt werden oral verabreichbare pharmazeutische Zusammensetzungen. in denen die aufgezeigten Kombinationen enthalten sind.

Zur Herstellung der pharmazeutischen Zubereitungen, die diese Kombinationen enthalten, werden die Wirkstoffe in den angegebenen Mengen mit physiologisch verträglichen Trägerstoffen und/oder Verdünnungsmitteln und/oder Hilfsstoffen in der gewünschten Weise formuliert.

Beispiele für die Träger- und Hilfsstoffe sind Gelatine, natürliche Zucker wie Rohrzucker oder Milchzucker. Lecithin, Pektin, Stärke (zum Beispiel Maisstärke oder Amylose), Cyclodextrine und Cyclodextrinderivate, Dextran, Polyvinylpyrrolidon, Polyvinylacetat, Gummi arabicum, Alginsäure, Tylose, Talkum, Lycopodium, Kieselsäure, Calciumhydrogenphosphat, Cellulose, Cellulosederivate wie Methyloxypropylcellulose, Methylcellulose, Hydroxypropylmethylcellulose, Hydroxypropylmethylcellulose-phthalat, Fettsäuren mit 12 bis 22 C-Atomen, Emulgatoren, Öle und Fette, insbesondere auch pflanzliche Glycerinester und Polyglycerinester aus gesättigten Fettsäuren, ein- oder mehrwertige Alkohole und Polyglykole wie Polyethylenglykole, Ester von aliphatischen gesättigten oder ungesättigten Fettsäuren 2 bis 22 C-Atome, mit einwertigen aliphatischen Alkoholen 1 bis 20 C-Atome oder mehrwertigen Alkoholen wie Glykolen, Glycerin, Diethylenglykol, Propylenglycol 1,2, Sorbit, Mannit.

Als weitere Hilfsstoffe kommen auch Stoffe in Frage, die den Zerfall bewirken (sogenannte Sprengmittel), vernetztes Polyvinylpyrrolidon, Natriumcarboxymethylstärke, Natriumcarboxymethylcellulose, mikrokristalline Cellulose. Ebenfalls können bekannte Hüllstoffe verwendet werden. Polymerisate sowie Copolymerisate der Acrylsäure und/oder Methacrylsäure und/oder deren Ester, Zein, Ethylcellulose, Ethylcellulosesuccinat, Schellack.

Als Plastifizierungsmittel für Hüllstoffe können in Frage kommen:
Citronen und Weinsäureester, Glycerin und Glycerinester, Polyethylenglycol verschiedener Kettenlängen. Zur Herstellung von Lösungen oder Suspensionen kommen beispielsweise Wasser oder physiologisch verträgliche organische Lösungsmittel in Frage , wie Alkohole und Fettalkohole.

Für flüssige Zubereitungen kann der Einsatz von Konservierungsstoffen wie Kaliumsorbat, Methyl-4-hydroxybenzoat oder Propyl-4-hydroxybenzoat, von Antioxidantien wie Ascorbinsäure und von Geschmacksverbesserern wie Pfefferminzöl erforderlich sein.

Bei der Herstellung der Zubereitungen können bekannte und übliche Lösungsvermittler, beziehungsweise Emulgatoren, wie Polyvinylpyrrolidon und Polysorbat 80 verwendet werden.

Für weitere Beispiele für infragekommende Träger- und Hilfsstoffe siehe auch Dr. H. P. Fiedler "Lexikon der Hilfsstoffe für Pharmazie, Kosmetik und angrenzende Gebiete".

In pharmazeutischen Zubereitungen, welche die Kombinationen aus prokinetisch wirksamen Antiemetika und Tramadol enthalten, sollte das Verhältnis 1 : 4 bis 1 : 10 betragen. Diese pharmazeutischen Zubereitungen enthalten als Einzeldosen im allgemeinen 5 - 80 mg eines Antiemetikums oder eines seiner Salze und 50 - 400 mg Tramadol oder eines seiner Salze. Dabei sollte die Tagesdosis vorzugsweise 20 - 80 mg Antiemetikum und 200 - 400 mg Tramadol enthalten.

Die genannten Tagesdosen können in Abhängigkeit von der therapeutischen Indikation entweder in Form einer einmaligen Verabreichung der gesamten Menge oder in Form von 2 bis 4 Teildosen pro Tag eingesetzt werden. Im allgemeinen ist eine Verabreichung 2 bis 4 mal täglich bevorzugt.

Die folgenden Beispiele sollen die Erfindung weiter erläutern und stellen damit keine abschließende Aufzählung dar.

### Beispiel 1

### Herstellung einer Metoclopramid - Lösung

802,4 g gereinigtes Wasser werden in einem geeigneten Behälter vorgelegt und unter Rühren werden 4,7 g Metoclopramidhydrochlorid-1-Wasser, 0,1 g Ascorbinsäure, 170,1 g Sorbit, 2,8 g Kaliumsorbat und eine Vorlösung aus 18,9 g Ethanol 96% (v/v), 0,7 g Methyl-4-hydroxybenzoat und 0,3 g Propyl-4-hydroxybenzoat zugegeben und gerührt, bis alle Bestandteile gelöst sind. Die Lösung wird durch einen geeigneten Filter filtriert.

| Rezeptur | Gewichtsteile (%) |
|---|---|
| Metoclopramidhydrochlorid-1-Wasser | 0,47 |
| Ascorbinsäure | 0,01 |
| Sorbit | 17, 01 |
| Kaliumsorbat | 0,28 |
| Ethanol 96% (v/v) | 1,89 |
| Methyl-4-hydroxybenzoat | 0,07 |
| Propyl-4-hydroxybenzoat | 0,03 |
| Gereinigtes Wasser | 80,24 |

### Abfüllung der Lösung:

Die Lösung wird auf einer geeigneten Abfüllmaschine in geeignete Tropfflaschen abgefüllt.

### Beispiel 2

### Herstellung der Tramadol - Lösung

484,2 g gereinigtes Wasser werden in einem geeigneten Behälter vorgelegt und unter Rühren werden 100,0 g Tramadolhydrochlorid, 1,5 g Kaliumsorbat, 161,8 g Ethanol 96% (v/v), 124,5 g Propylenglycol 1,2, 200,0 g Kristallzucker, 1,0 g Polysorbat 80 und 1,0 g Pfefferminzöl zugegeben und gerührt, bis alle Bestandteile gelöst sind. Die Lösung wird durch einen geeigneten Filter filtriert.

| Rezeptur | Gewichtsteile (%) |
|---|---|
| Tramadolhydrochlorid | 9,3 |
| Kaliumsorbat | 0,1 |
| Ethanol 96% (v/v) | 15,1 |
| Propylenglycol 1,2 | 11,6 |
| Kristallzucker | 18,6 |
| Polysorbat 80 | 0,1 |
| Pfefferminzöl | 0,1 |
| Gereinigtes Wasser | 45,1 |

### Abfüllung der Lösung:

Die Lösung wird auf einer geeigneten Abfüllmaschine in geeignete Tropfflaschen abgefüllt.

### Beispiel 3

### Tramadol - Metoclopramid - Retardpellets

### Herstellung der wirksoffhaltigen Kerne

Auf 1000 g Neutralpellets geeigneter Größe (z. B. mit einem Durchmesser zwischen 0,5 und 0,6 mm) wird mit ca. 2200 g einer 15 %igen Lösung von Ethylcellulose/Schellack (6 : 4) in einem Ethanol-Wasser-Gemisch ca. 96 % [V/V] 4824 g Tramadolhydrochlorid-Metoclopramidhydrochlorid-1-Wasser-Aerosil® 200-Gemisch im Drageekessel aufgebracht. Die erhaltenen Kerne werden anschließend getrocknet und gesiebt (0,8 - 1,4 mm).

### Aufbringung der Membran

Auf 6,15 kg der so hergestellten wirkstoffhaltigen Kerne wird eine Membran aufgebracht, indem 470 g einer 15 %igen Lösung von Ethycellulose/Schellack (6 : 4) in einem Ethanol-Wasser-Gemisch ca. 96 % [V/V] aufgegeben werden. Als Trennmittel werden 700 g Talkum eingepudert.

| Rezeptur | Gewichtsteile (%) |
|---|---|
| Tramadolhydrochlorid | 57,8 |
| Metoclopramidhydrochlorid-1-Wasser | 11,6 |
| Neutralpellets | 14,4 |
| Ethylcellulose | 3,5 |
| Schellack | 2,3 |
| Aerosil 200 | 0,3 |
| Talkum | 10,1 |
| Ethanol-Wasser-Gemisch ca. 96 % [V/V] | q.s. |

### Wirkstoff-Freisetzung

Die in-vitro-Freisetzung von Tramadolhydrochlorid aus den Retardpellets gemäß Beispiel 3 wird nach USP 23/NF 18 in Apparat 3 bestimmt. Die Temperatur des Freisetzungsmediums beträgt 37° C, die Hubzahl der Probenröhren 20 Hübe/Minute und die Menge Testlösung pro Untersuchungsintervall 175 ml.

Die Untersuchung wird mit Testlösung pH 1.5 begonnen, nach der ersten Stunde wechseln die Röhren mit den Proben in jeweils 175 ml Testlösung pH 4.5, nach der 2. Stunde in eine Testlösung pH 6.9, nach der 4. Stunde in eine neue Testlösung pH 6.9, nach der 6. Stunde in Testlösung pH 7.2 und nach der 8. Stunde in eine Testlösung pH 7.5. Die zu den vorgenannten Zeitpunkten im Lösungsmedium befindliche freigesetzte Wirkstoffmenge wird spektralphotometrisch bestimmt. Es werden folgende Freisetzungswerte für Tramadolhydrochlorid ermittelt:

| Zeit in Stunden | Freigesetzter Anteil in Gew.- % |
|---|---|
| 1 | 39 |
| 2 | 57 |
| 4 | 70 |
| 6 | 78 |
| 8 | 84 |
| 12 | 93 |

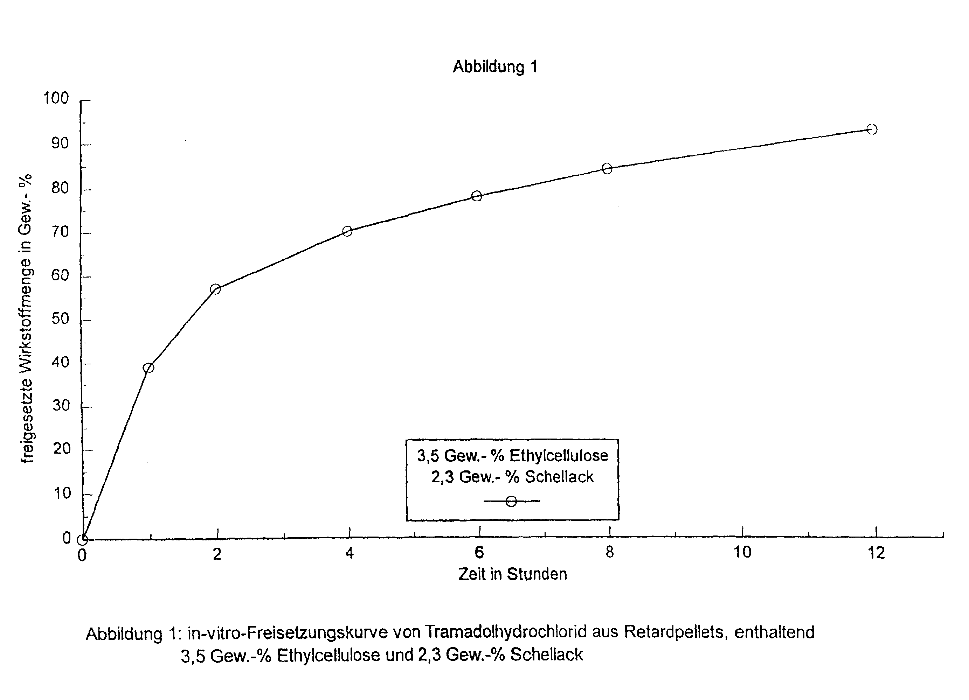

Die in-vitro-Freisetzungskurve der Retardpellets ist in Abbildung 1 dargestellt.

### Beispiel 4

### Tramadol - Metoclopramid - Kapsel

### Herstellung der Kapselfüllmasse

323 g Tramadolhydrochlorid, 6,5 g Metoclopramidhydrochlorid-1-Wasser, 597 g Calciumhydrogenphosphat, 0,5 g Aerosil® 200 und 1,0 g Magnesiumstearat werden gesiebt und in einem geeigneten Mischer gemischt.

| Rezeptur | Gewichtsteile (%) |
|---|---|
| Tramadolhydrochlorid | 32,3 |
| Metoclopramidhydrochlorid-1-Wasser | 6,5 |
| Calciumhydrogenphosphat | 59,7 |
| Aerosil® 200 | 0,5 |
| Magnesiumstearat | 1,0 |

### Herstellung der Kapseln:

Die Kapselfüllmasse wird mit dem Sollfüllgewicht von 155 mg auf einer geeigneten Kapselfüllmaschine in Hartgelatinekapseln geeigneter Größe abgefüllt.

### Beispiel 5

### Tramadol + Metoclopramid - Trinktablette

### Herstellung der Pulvermischung:

251 g Tramadolhydrochlorid, 25 g Metoclopramidhydrochlorid 1H₂O, 4 g Aerosil® 200, 50 g Aspartam®, 100 g Crospovidon, 700 g mikrokristalline Cellulose, 819,5 g Lactose Monohydrat, 37,5 g Aroma (z. B. Erdbeere), 10 g Natriumdodecylsulfat und 3 g Magnesiumstearat werden gesiebt und in einem geeigneten Mischer gemischt.

| Rezeptur | Gewichtsteile (%) |
|---|---|
| Tramadolhydrochlorid | 12,55 |
| Metoclopramidhydrochforid-1-Wasser | 1,25 |
| Aerosil® 200 | 0,20 |
| Aspartam® | 2,50 |
| Crospovidon | 5,00 |
| Mikrokristalline Cellulose | 35,00 |
| Lactose | 40,98 |
| Aroma | 1,88 |
| Natriumdodecylsulfat | 0,50 |
| Magnesiumstearat | 0,15 |

### Herstellung der Tabletten:

Die Pulvermischung wird durch Verwendung einer geeigneten Tablettiermaschine in Tabletten verpreßt (400 mg nominal).

## Patentansprüche

1. Verwendung pharmazeutischer Kombinationen aus mindestens einem prokinetisch wirksamen Antiemetikum oder pharmazeutisch akzeptable Salzen davon und Tramadol, seinen Enantiomeren oder pharmazeutisch akzeptablen Salzen zur Herstellung eines Arzneimittels zur Behandlung der Migräne oder migräniformen Kopfschmerzen.

2. Verwendung nach Anpruch 1 **dadurch gekennzeichnet,**
**daß** die pharmazeutischen Kombinationen als Antiemetikum Metoclopramid, Domperidon oder 5-HT₃-Antagonisten enthalten.

3. Verwendung nach Anpruch 1 **dadurch gekennzeichnet,**
**daß** die pharmazeutischen Kombinationen 5 - 80 mg eines Antiemetikums oder eines seiner Salze und 50 - 400 mg Tramadol oder eines seiner Salze enthalten.

4. Verwendung nach Anpruch 1 **dadurch gekennzeichnet,**
**daß** die pharmazeutische Kombinationen vorzugsweise oral verabreicht werden.

## Claims

1. Use of pharmaceutical combinations of at least one prokinetically active antiemetic or pharmaceutically acceptable salts thereof and tramadol, its enantiomers or pharmaceutically acceptable salts for the production of a medicament for the treatment of migraine or migrainoid headaches.

2. Use according to Claim 1, **characterized in that** the pharmaceutical combinations contain metoclopramide, domperidone or 5-HT₃ antagonists as antiemetic.

3. Use according to Claim 1, **characterized in that** the pharmaceutical combinations contain 5-80 mg of an antiemetic or of one of its salts and 50-400 mg of tramadol or one of its salts.

4. Use according to Claim 1, **characterized in that** the pharmaceutical combinations are preferably administered orally.

## Revendications

1. Utilisation de combinaisons pharmaceutiques. constituées d'au moins un antiémétique actif en tant que procinétique ou des sels pharmaceutiquement acceptables de celui-ci et de Tramadol, ses énantiomères ou ses sels pharmaceutiquement acceptables pour la préparation d'un médicament pour le traitement de la migraine ou de céphalées migraineuses.

2. Utilisation selon la revendication 1, **caractérisée en ce que** les combinaisons pharmaceutiques contiennent, en tant qu'antiémétique du Metoclopramide, du Domperidone ou des agonistes de 5-HT₃.

3. Utilisation selon la revendication 1, **caractérisée en ce que** les combinaisons pharmaceutiques contiennent 5 - 80 mg d'un antiémétique ou d'un de ses sels et 50 - 400 mg de Tramadol ou un de ses sels.

4. utilisation selon la revendication 1, caracté risée en ce que les combinaisons pharmaceutiques sont de préférence administrées par voie orale.
